# EUROPÄISCHE PATENTSCHRIFT

(11) **EP 1 686 912 B1**
(45) Veröffentlichungstag und Bekanntmachung des Hinweises auf die Patenterteilung: **25.01.2017**
(21) Anmeldenummer: 04802673.6
(22) Anmeldetag: 05.11.2004
(51) Int. Cl.: A61B 34/30

(54) **AKTORPLATTFORM ZUR FÜHRUNG VON ENDEFFEKTOREN BEI MINIMAL INVASIVEN INTERVENTIONEN**
ACTUATOR PLATFORM FOR GUIDING END EFFECTORS IN MINIMALLY INVASIVE INTERVENTIONS
PLATEFORME ACTRICE DESTINÉE À GUIDER DES EFFECTEURS TERMINAUX LORS D'INTERVENTIONS À INVASION MINIMALE

(30) Priorität: 12.11.2003 DE 10353110
(43) Veröffentlichungstag der Anmeldung: 09.08.2006
(73) Patentinhaber: Micro-Epsilon Messtechnik GmbH & Co. KG, 94496 Ortenburg (DE)
(72) Erfinder: FEUSSNER, Hubertus, 81671 München (DE); SAMMEREIER, Eduard, 94542 Haarbach/Rainding (DE); SELLEN, Martin, 94496 Ortenburg (DE); KIRSCHENHOFER, Ludwig, 93047 Regensburg (DE)
(74) Vertreter: Glück Kritzenberger Patentanwälte PartGmbB
(86) Internationale Anmeldenummer: PCT/DE2004/002442
(87) Internationale Veröffentlichungsnummer: WO 2005/046499

(56) Entgegenhaltungen:
- WO-A-01/89405
- US-A- 5 078 140
- US-A- 5 876 325
- US-A- 5 971 976
- US-A- 6 001 108
- US-A1- 2001 013 764
- US-B1- 6 547 782

## Beschreibung

Die Erfindung bezieht sich auf einen Roboter bzw. auf eine universelle Aktorplattform zur Führung von Endeffektoren, beispielsweise Kameras, Werkzeugen usw., die für minimal invasive Interventionen in den Körperraum eines menschlichen oder tierischen Körpers an einer Eintritts- oder Operationsöffnung eingeführt sind. Operations-Assistenz -Roboter oder Aktorplattformen zur Verwendung bei medizinischen Interventionen bzw. Operationen sind z.B. zur Führung von Hilfsinstrumenten, wie z.B. Kameras usw. grundsätzlich bekannt.

Bekannt sind auch minimal invasive Interventionen, bei denen ein Instrument, beispielsweise chirurgisches Instrument oder ein optisches oder bildgebendes Instrument, beispielsweise ein Endoskop, über eine kleinformatige Operationsöffnung in den Innenraum eines Patientenkörpers eingeführt wird.

Das Dokument US-A-6001108 zeigt eine Aktorplattform mit den in der Preambel des Anspruchs 1 definierten Merkmalen.

Aufgabe der Erfindung ist es, ein universelles Robotersystem bzw. eine universelle Aktorplattform zum Führen von Endeffektoren bei minimal invasiven Interventionen aufzuzeigen, die (Aktorplattform) den vielfältigen und sich teilweise auch widersprechenden Anforderungen des täglichen klinischen Einsatzes voll gerecht wird, und die u.a. auch bei kleiner und kompakter Bauform ein geringes Gewicht bei hoher Steifigkeit aufweist, die die Verwendung von bildgebenden Systemen verschiedenster Art für Untersuchungen und/oder Überwachungen innerhalb des menschlichen oder tierischen Körpers ungestört ermöglicht und deren Antriebe und Steuerungen eine präzise Bewegung der Endeffektoren selbst bei äußeren Störeinflüssen sicherstellen. Zur Lösung dieser Aufgabe ist eine Aktorplattform entsprechend dem Patentanspruch 1 ausgebildet.

"Bildgebende oder -erzeugende Medien" sind entsprechend der Erfindung beispielsweise Röntgenstrahlung, Magnetfelder oder elektromagnetische Wellen von auf diesen Medien basierenden, im medizinischen Bereich verwendeten bildgebenden Verfahren oder Systemen, wie z.B. Röntgengeräte, auf Röntgenstrahlung, Kernspin oder Magnetoresonanz basierende Computer-Tomographie-Systeme oder - Einrichtungen, elektromagnetische Positions-Bestimmungs-Einrichtungen oder - Systeme usw..

Ein "neutraler Werkstoff" im Sinne der Erfindung ist ein Werkstoff, welcher für diese bildgebenden oder -erzeugenden Medien neutral oder annähernd neutral ist, d.h. insbesondere für das betreffende Medium durchlässig ist und keine oder zumindest keine wesentliche Reaktion mit dem bildgebenden oder -erzeugenden Medium zeigt. Neutrale Werkstoffe in diesem Sinne sind unter anderem Materialien, die weder ferromagnetisch, noch diamagnetisch und auch nicht elektrisch leitend sind bzw. eine nur geringe elektrische Leitfähigkeit und bevorzugt auch nur geringe dielektrische Verluste aufweisen.

Als neutrale Werkstoffe eignen sich beispielsweise Kunststoffe, aber auch anorganische Werkstoffe, wie z.B. Keramik, eventuell auch Legierungen weicher Metalle, z.B. Aluminiumlegierungen.

Wird das erfindungsgemäße Operations-Assistenz-System an einer Operationsliege verwendet, so sind bevorzugt zumindest solche Elemente des Systems, und zwar einschließlich der Antriebe, Gelenke usw., aus einem oder aus mehreren neutralen Werkstoffen gefertigt, die (Elemente) sich oberhalb der Operationstischebene befinden.

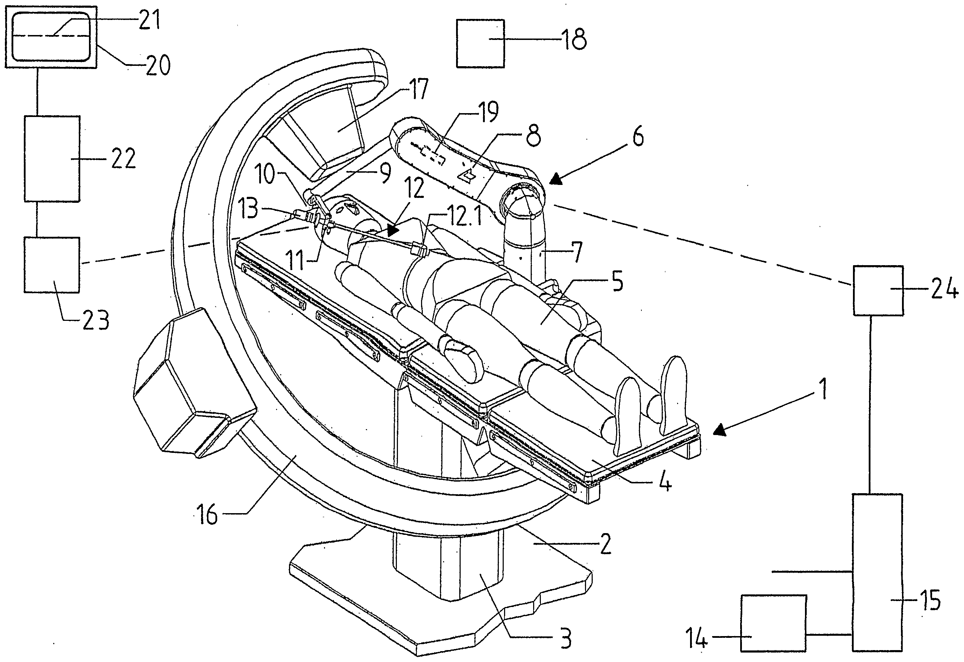

Die Erfindung wird im Folgenden anhand der Figur an einem Ausführungsbeispiel näher erläutert. Die Figur zeigt in vereinfachter Darstellung einen Operationstisch mit einem C-Bogen mit bildgebender Einrichtung und mit einer Aktorplattform für das Führen von Endeffektoren bei einer minimal invasiven Intervention.

Der in der Figur allgemein mit 1 bezeichnete Operationstisch besteht in bekannter Weise aus einem Fußteil 2, einer Hubsäule 3 und dem eigentlichen Tischelement 4, welches die Auflage für den Patienten 5 während einer Operation bzw. minimal invasiven Intervention bildet.

Mit dem Operationstisch 2 verbunden ist bei der dargestellten Ausführungsform eine in der Figur allgemein mit 6 bezeichnete Kinematik einer universellen Aktorplattform (Robotersystem). Bei der dargestellten Ausführungsform besteht die Kinematik u.a. aus einer Tragsäule 7 und aus mehreren Armen 8, 9 und 10, von denen der Arm 10 an seinem freien Ende eine Schnittstelle oder Aufnahme 11 bildet, an der ein Endeffektor 12, welcher bei der dargestellten Ausführungsform ein Endoskop mit Kamera 13 ist, um mehrere Achsen motorisch beweglich gehalten ist. Die Kinematik 6 bildet mehrere Bewegungsachsen, um die bzw. in denen die Aufnahme 11 schwenkbar bzw. bewegbar ist.

Der Endeffektor 12 ist für die Operation (minimal invasive Intervention) mit seinem Kopf bzw. mit seiner Instrumenten- oder Endeffektorspitze 12.1 (beispielsweise Optik des Endoskops) durch eine Operationsöffnung in den Operationsbereich im Körper des Patienten 5 eingeführt und kann mit entsprechenden Antrieben der Aktorplattform bzw. Kinematik 6 durch den Operateur gesteuert bewegt werden, und zwar über eine Eingabeeinrichtung 14 beliebiger Ausbildung einer Antriebssteuerung oder Steuerelektronik 15. Mit der Steuerelektronik 15, die Teil der Aktorplattform ist, wird die Kinematik derart gesteuert, dass die Instrumenten- oder Endeffektorspitze 12.1 in der gewünschten Weise im Körperraum des Patienten 5 bewegt wird, ohne dass der Endeffektor-Bereich an der Operationsöffnung als invarianter Punkt bei dieser Bewegung seine Lage verändert oder nennenswert verändert.

Am Operationstisch 1 befindet sich weiterhin ein sogenannter C-Bogen 16, an welchem eine bildgebende Einrichtung 17, beispielsweise die Strahlungsquelle einer bildgebenden Einrichtung auf Röntgenbasis vorgesehen ist, und zwar für eine bildgebende Untersuchung und/oder Überwachung des Operationsbereichs des Patienten.

Als bildgebende Einrichtungen bzw. Methoden kommen aber außer solchen auf Röntgenbasis auch andere bildgebende Verfahren oder Systeme in Frage, wie beispielsweise Kernspin, Magnetresonanz usw.. Weiterhin besteht die Möglichkeit, zur genauen Bestimmung und/oder Überprüfung der Position der I Instrumenten- oder Endeffektorspitze 12.1 eine elektromagnetische Positionsbestimmungseinrichtung 18 vorzusehen.

Damit eine ungestörte bildgebende Untersuchung bei allen üblicherweise verwendeten Verfahren und Systeme möglich ist, bestehen sämtliche Funktionselemente der Kinematik 6 zumindest oberhalb der Ebene des Tischelementes 4, insbesondere sämtliche Arme 8 - 10, Gelenke sowie Antriebselemente aus einem für die verwendeten bildgebenden Verfahren oder Systeme bzw. deren Medien neutralen Material, d.h. insbesondere nicht aus einem elektrisch nichtleitenden Werkstoff und auch nicht aus einem ferro- oder diamagnetischem Material.

Geeignete Werkstoffe sind beispielsweise isolierende und zugleich auch magnetisch neutrale Werkstoffe, wie beispielsweise Kunststoff mit ausreichender Festigkeit, z.B. PA (Polyamid), POM oder PE (Polyethylen). Bedingt geeignet sind auch Aluminiumlegierungen.

Ungeeignet für die Funktionselemente der Kinematik 6 zumindest oberhalb der Ebene des Tischelementes 4 sind auf jeden Fall alle magnetischen und/oder metallische Werkstoffe, wie Stähle, insbesondere auch rostfreie Stähle, Werkstoffe mit hoher Dichte.

Als Antriebs- bzw. Stellglieder eignen sich bei der Erfindung beispielsweise Stellzylinder, z.B. hydraulische Stellzylinder, wie dies in der Figur mit 19 angedeutet ist. Diese Stellglieder bestehen dann ebenfalls aus einem für das bildgebende Medium neutralen Material.

Die Instrumenten- oder Endeffektorspitze 12.1 ist beispielsweise so ausgebildet, dass sie von dem bildgebenden System erfasst wird, so dass über das bildgebende System die Lage dieser Spitze ebenfalls angezeigt wird.

Nachstehend wird auf weitere, spezielle Merkmale der universellen Aktorplattform 6 bzw. deren Bestandteile eingegangen.

### Kinematik 6

Die Kinematik 6 ist so ausgeführt, dass sie eine Bewegung des Endeffektors 12 bzw. des Kopfes oder der Spitze 12.1 um 360° um den von der jeweiligen Operationsöffnung gebildeten Eintrittspunkt des Endeffektors 12 in den KörperInnenraum des Patienten 5 ermöglicht, und zwar, wie bereits ausgeführt, ohne dass sich die Lage desjenigen Bereichs des Endeffektors 12 verändert oder aber wesentlich verändert, an dem (Bereich) dieser Endeffektor 12 am Eintrittspunkt bzw. an der Operationsöffnung in den Körper eingeführt ist. Weiterhin ist die Kinematik 6 so ausgeführt, dass sie eine Neigung des Endeffektors 12 um mindestens 75° zur Lotrechten auf die Eintrittsfläche ermöglicht, an der die Operationsöffnung bzw. Eintrittsöffnung vorgesehen ist.

Neben ihrer Herstellung aus den neutralen Werkstoffen wird von der Kinematik 6 weiterhin gefordert, dass sie klein und kompakt ausgeführt ist und den Raum um den Operationstisch 1 bzw. das Tischelement 4 nicht verengt, d.h. der Platzbedarf für die Kinematik 6 auch bei extremen Bewegungen im Wesentlichen nur dem Platzbedarf eines menschlichen Operateurs entspricht, bevorzugt aber kleiner ist als der Raum, der ein menschlicher Operateur beanspruchen würde.

Weiterhin wird für die Kinematik ein geringes Gewicht gefordert, d.h. ein Gesamtgewicht kleiner als 15 kg, sodass die Kinematik problemlos und bequem am Tischelement 4, oder an dortigen Befestigungsschienen (z.B. Rails) gegebenenfalls auch unter Verwendung von Schnellverschlüssen oder Schnellspannern befestigt und auch vom Operationstisch wieder abgenommen werden kann.

Ein weiteres wesentliches Merkmal der Kinematik 6 besteht auch darin, dass sie in einem Notfall schnell entriegelbar ist, sodass es dann möglich ist, den jeweiligen Endeffektor 12 manuell aus dem Körper des Patienten 5 zu entfernen. Die Entriegelung erfolgt beispielsweise an wenigstens einem Gelenk zwischen zwei benachbarten Armen der Kinematik 6.

Um eine wirksame Reinigung und Sterilisierung der Kinematik 6 zu ermöglichen, weist diese eine entsprechende, geschlossene Bauweise auf, die z.B. dadurch erreichbar ist, dass die Außenfläche der Kinematik zumindest im Bereich der Gelenke von einem flexiblen Schlauch gebildet ist.

Die Antriebe für die Kinematik 6 sind vorzugsweise fluidische, z.B. hydraulische Antriebe, die bei kleiner Baugröße hohe Kräfte und Momente ermöglichen und insbesondere auch eine hohe Steifigkeit der Kinematik 6 gewährleisten. Weiterhin machen die Fluid-Antriebe auch langsame und präzise Bewegungen möglich.

Um die Kinematik 6 bei Systemen einsetzen zu können, die auf Magnetresonanz und/oder Röntgenstrahlen und/oder elektromagnetischen Feldern basieren, sind die in der Kinematik vorhandenen Antriebe vorzugsweise Sekundärantriebe oder Nehmer, die von Primärantrieben oder Gebern außerhalb des Einflussbereichs der Magnetresonanz, Röntgenstrahlen oder elektromagnetischen Feldern angeordnet sind. Die Geberantriebe sind beispielsweise Pumpen oder Geberzylinder. Die Nehmerantriebe sind beispielsweise Zylinder.

### Antriebssteuerung oder Steuerelektronik 15

Bestandteil der universellen Aktorplattform ist weiterhin die Steuerelektronik 15, die über die Eingabe 14 (für Sollwertbildung) eine Steuerung der Kinematik 6 auch in kleinen Schritten und sehr einfach ermöglicht. Die Eingabe 14 kann beliebig ausgebildet sein und ermöglicht beispielsweise eine manuelle Steuerung oder manuelle Eingabe von Steuerbefehlen. Grundsätzlich besteht die Möglichkeit, diese Eingabe 14 an medizinischen Werkzeugen vorzusehen, die zusätzlich zu dem an der Kinematik 6 angebrachten Endeffektor 12 vom Operateur verwendet wird. Auch andere Steuerungen bzw. Eingaben sind möglich, z.B. eine automatische Steuerung über bildgebende Elemente oder Systeme und/oder über Sensorelemente (z.B. an dem Kopf 12.1 des Endeffektors 12). Auch eine Sprachsteuerung ist möglich.

Die über die Eingabe 14 eingegebenen Werte werden dann beispielsweise als Sollwerte mit von Sensoren gelieferten Istwerten, die den jeweiligen aktuellen Zustand bzw. die jeweilige aktuelle Stellung der Kinematik 6 definieren, verglichen, sodass dann durch einen geschlossenen Regelkreis eine sehr exakte Positionierung und Bewegung des Endeffektors 12 möglich ist.

Die Steuerelektronik 15 zeichnet sich vorzugsweise auch noch durch weitere Merkmale aus. So wird durch die Steuerelektronik, gegebenenfalls im Zusammenwirken mit äußeren Sensoren auch eine Störgrößenkompensation bei der Steuerung der Kinematik 6 erreicht, und zwar insbesondere eine Kompensation von äußeren mechanischen Stößen oder Vibrationen am Operationstisch 1 sowie eine Kompensation von elektrischen und elektromagnetischen Störgrößen und/oder Temperatureinflüssen.

Weiterhin erfolgt die Steuerung der Kinematik 6 durch die Steuerelektronik 15 in der Weise, dass vor der Einleitung einer Bewegung oder Positionsänderung zunächst eine Plausibilitätsprüfung erfolgt, z.B. durch ein Vergleichen der aktuellen Position des Endeffektors 12 oder des Kopfes 12.1 mit der jeweiligen Eingabe, wobei auch Hindernisse selbsttätig erkannt und beispielsweise durch Bewegen des Endeffektors 12 auf "Umwegen" umgangen werden. Weiterhin erfolgt grundsätzlich eine Begrenzung des Bewegungsraumes des Endeffektors 12 bzw. des Kopfes 12.1.

Bei einer bevorzugten Ausführungsform der Erfindung ist die Steuerelektronik so ausgeführt, dass eine Nachführungsoptimierung der Kinematik 6 im Stand erfolgt. Hierbei wird durch Bewegen der Kinematik ohne ein Bewegen des Endeffektors 12, d.h. unter Beibehaltung der aktuellen Stellung dieses Endeffektors der Zustand der einzelnen Bewegungsachsen der Kinematik 6 so optimiert, dass aus dem dann erreichten Zustand jede Bewegungsachse die ihr zugeordneten Bewegungen bei einem entsprechenden Befehl ohne Beschränkungen ausführen kann, d.h. dass sich beispielsweise keine Bewegungsachse in einer Endlage befindet.

Weiterhin ist die Steuerelektronik bevorzugt ausgebildet, dass die durch eine Eingabe veranlasste Bewegung des Endeffektors 12 auf einer optimalen, möglichst kurzen Bewegungsbahn und/oder innerhalb eines kleinen Bewegungsraumes der Kinematik erfolgt, sodass auch hierdurch der Platzbedarf für die Kinematik 6 klein gehalten wird.

Über anpassbare Schnittstellen ist die Steuerelektronik 15 mit weiteren externen Einrichtungen verbindbar, beispielsweise für eine Sprachsteuerung usw..

Bei der dargestellten Ausführungsform ist das Instrument 12 mit der Kamera 13 frei oder weitestgehend frei drehbar um die Instrumentenlängsachse an dem freien Ende des Armes 10 bzw. des Instrumentenhalters 11 gehalten, sodass sich das beispielsweise schräg gestellte Instrument 12 bei einem Schwenken um die Achse des invarianten Punktes bzw. der Körperöffnung, an dem bzw. an der das Instrument 12 in den Körper des Patienten 5 eingeführt ist, mit seinem Umfang an dem Rand der Körperöffnung abwälzt und somit um seine Achse dreht. Dies führt dazu, dass sich auch das mit der Kamera 13 erfasste und an einem Monitor 20 wiedergegebene Kamerabild bzw. dessen in der Figur mit 21 angedeutete Bildhorizont drehen würden und dadurch die visuelle Auswertung des Kamerabildes für den Operateur äußerst schwierig wäre.

Um dies zu vermeiden, wird das von der Kamera 13 gelieferte Bild in eine Bildbearbeitung 22 so bearbeitet, dass auch bei einem Schwenken des Instrumentes 12 um die Achse der Körperöffnung und bei dem damit verbundenen Drehen des Instrumentes 12 um seine Instrumentenachse zumindest die Ausrichtung des Bildes bzw. des Bildhorizontes 21 unverändert oder im Wesentlichen unverändert bleiben. Hierfür ist die Bildverarbeitung 22, die selbstverständlich auch Bestandteil der Steuerelektronik 15 bzw. eines entsprechenden Rechners oder einer dortigen Software sein kann, mit einem Sensor 23 verbunden, der am Arm 10 bzw. am Instrumentenhalter 11 vorgesehen ist und ein Sensorsignal liefert, welches der Drehstellung des Instrumentes 12 bzw. der Kamera 13 um die Instrumentenachse relativ zum Arm 10 entspricht. Mit diesem Sensorsignal erfolgt dann eine Bearbeitung bzw. ein Drehen des von der Kamera 13 gelieferten Bildes in der Weise, dass zumindest der Bildhorizont 21 seine Orientierung beibehält.

Weiterhin ist es möglich, diese Korrektur durch eine intelligente Bildverarbeitung vorzunehmen, und zwar derart, dass eine Lagekorrektur des von der Kamera 13 gelieferten Bildes in der Bildverarbeitung 22 beispielsweise anhand von markanten Bildbestandteilen und/oder aber anhand von in der Kamera erzeugten zusätzlichen Bildpunkten erfolgt, die eine feste vorgegebene Lage in der Bildebene der Kamera 13 aufweisen.

Da die Kinematik 6 Sensoren aufweist, mit denen die jeweilige Stellung der Kinematik 6 sowie deren die Bewegung in bzw. um die Kinematik-Achsen erfasst werden und hieraus u.a. auch die Lage und Orientierung des Instrumentes 12 und der Kamera 13 z.B. von der Steuereinrichtung 15 errechnet werden können, besteht als weitere Möglichkeit zur Beibehaltung zumindest der Orientierung des Bildhorizontes 21, das von der Kamera 13 gelieferte Bild in Abhängigkeit der von diesen Sensoren gelieferten Signale zu korrigieren. Diese Sensoren sind in der Figur schematisch mit 24 angegeben.

Sind die Stellzylinder 19, wie bereits erwähnt, Nehmerzylinder jeweils eines Nehmer-Geber-Systems, bei dem jeder Stellzylinder 19 durch einen Steuerzylinder über eine Fluidverbindung (hydraulische Verbindung) angesteuert wird, so befinden sich die Sensoren 24 ebenso wie die Steuerzylinder außerhalb des Einflussbereichs der Magnetresonanz, Röntgenstrahlen und/oder elektrischen Bilder.

Vorstehend wurde davon ausgegangen, dass zur Beibehaltung der Orientierung des Bildhorizontes 21 das von der Kamera 13 gelieferte Bild oder Bildsignal korrigiert wird. Es besteht weiterhin aber auch die Möglichkeit, die Beibehaltung der Orientierung des Bildhorizontes 21 dadurch zu erreichen, dass durch einen Stellantrieb die Kamera 13 beispielsweise zusammen mit dem Instrument 12 um die Achse dieses Instrumentes gedreht wird, und zwar vorzugsweise automatisch z.B. unter Verwendung der von den Sensoren 24 der Kinematik gelieferten Signale.

Die Erfindung wurde voranstehend an einem Ausführungsbeispiel beschrieben. Es versteht sich, dass zahlreiche Änderungen sowie Abwandlungen möglich sind, ohne dass dadurch der der Erfindung zugrunde liegende Erfindungsgedanke verlassen wird.

### Bezugszeichenliste

- 1: Operationstisch
- 2: Fußteil
- 3: Hubsäule
- 4: Tischelement
- 5: Patient
- 6: Kinematik
- 7: Tragsäule
- 8 - 10: Arm
- 11: Instrumentenhalter
- 12: Instrument
- 12.1: Optik
- 13: Kamera
- 14: Eingabe
- 15: Steuerelektronik
- 16: C-Bogen
- 17: Element oder Strahlungsquelle für bildgebende Einrichtung
- 18: elektromagnetische Positionserfassungseinrichtung, Stellglied
- 19: Stellzylinder
- 20: Monitor
- 21: Bildhorizont
- 22: Bildbearbeitung
- 23,24: Sensor

## Patentansprüche

1. Universelle Aktorplattform zur Führung von Endeffektoren (12) bei minimal invasiven Interventionen, bei denen der jeweilige Endeffektor (12) an einem Eintrittspunkt in einen Körperinnenraum eingeführt ist,
mit einer wenigstens eine Schnittstelle zum Befestigen wenigstens eines Endeffektors (12) aufweisenden Kinematik (6),
mit wenigstens einem Antrieb (19) für die Kinematik (6) sowie mit einer Antriebssteuerung (15) zur Steuerung des Antriebs (19) der Kinematik (6), wobei die Kinematik (6) Mittel zum Befestigen an einem Operationstisch (1) oder dortigen Befestigungselementen, beispielsweise an Rails besitzt und eine geschlossene, sterilisierbare Bauform aufweist,
**dadurch gekennzeichnet,**
**dass** die Kinematik (6) für ein Neigen des Endeffektor (12) um wenigstens 75° bezogen auf eine Lotrechte auf die Ebene des Eintrittspunktes sowie für ein Bewegen des geneigten Endeffektors (12) um 360° um die Lotrechte auf die Ebene des Eintrittspunktes ausgebildet ist,
**dass** die Kinematik (6) zumindest in einem Teilbereich aus einem für das Medium einer bildgebenden und/oder die Positionen und/oder Orientierung bestimmenden Einrichtung systemneutralen Werkstoff besteht,
**dass** die Kinematik (6) manuell entriegel- oder trennbar ist, und
**dass** die Antriebssteuerung (15) zur Sollwertbildung eine Eingabe (14) besitzt und für eine Kompensation von mechanischen, elektrischen oder temperaturbedingten Störgrößen der Steuerung des Antriebs (19) der Kinematik (6) ausgebildet ist, indem die Antriebssteuerung (15) zur Erzielung einer hohen Genauigkeit und Sicherheit Teil eines geschlossenen Regelkreises ist, in welchem durch einen Vergleich des von wenigstens einem Sensor gelieferten und die tatsächliche Stellung der Kinematik (6) repräsentierenden Istwertes mit einem Sollwert eine Regelung der Positionierung des Antriebs (19) erfolgt.

2. Aktorplattform nach Anspruch 1, **dadurch gekennzeichnet, dass** bei Ausbildung des Endeffektors (12) als Kamera oder Teil einer Kamera und bei Ausbildung der Kinematik (6) für ein Drehen des geneigten Endeffektors (12) um seine Achse beim Bewegen um die Lotrechte auf die Ebene des Eintrittspunktes mechanische Mittel und/oder Software-Mittel (22), vorgesehen sind, um zumindest die Lage und/oder Orientierung eines auf einem Bildschirm (20) wiedergegebenen Kamerabildes konstant oder annähernd konstant zu halten.

3. Aktorplattform nach Anspruch 1 oder 2, **dadurch gekennzeichnet, dass** die Kinematik (6) an wenigstens einem Gelenk manuell entriegel- oder trennbar ist.

4. Aktorplattform nach einem der vorhergehenden Ansprüche, **dadurch gekennzeichnet, dass** die Kinematik (6) seitlich an einer Seite, beispielsweise an einer Längsseite des Operationstisches (1) oder an dortigen Befestigungselementen, beispielsweise Rails befestigbar ist.

5. Aktorplattform nach einem der vorhergehenden Ansprüche, **dadurch gekennzeichnet, dass** die Antriebssteuerung so ausgebildet ist, dass die Steuerung der Bewegung des wenigstens einen Endeffektors (12) durch die Antriebssteuerung (15) unter Berücksichtigung einer Plausibilitätsprüfung und/oder innerhalb eines begrenzten Bewegungsraumes erfolgt.

6. Aktorplattform nach einem der vorhergehenden Ansprüche, **dadurch gekennzeichnet,**
**dass** die Antriebssteuerung so ausgebildet ist, dass die Bewegung des wenigstens einen Endeffektors (12) durch die Antriebssteuerung (15) auf einer optimalen Bewegungsbahn, beispielsweise auf möglichst kurzen Wegen und/oder innerhalb eines möglichst kleinen Bewegungsraumes erfolgt,
und/oder
**dass** die Sollwert-Eingabe und/oder die Steuerung in kleinen Schritten und einfach
erfolgt.

7. Aktorplattform nach einem der vorhergehenden Ansprüche, **dadurch gekennzeichnet, dass** die Kinematik (6) bzw. deren Elemente (7, 8, 9, 10, 19) zumindest in einem oberhalb einer Ebene einer Patientenauflage (4) gebildeten Teil aus dem neutralen Werkstoff besteht.

8. Aktorplattform nach einem der vorhergehenden Ansprüche, **dadurch gekennzeichnet, dass** die Kinematik (6) mit wenigstens einem Schnellspannverschluss an dem Operationstisch (1) oder der Patientenauflage (4) befestigbar ist.

9. Aktorplattform nach einem der vorhergehenden Ansprüche, **dadurch gekennzeichnet, dass** der wenigstens eine Antrieb (19) der Kinematik (6) ein Fluid-Antrieb, vorzugsweise ein solcher mit wenigstens einem Geberelement und einem Nehmerelement ist.

10. Aktorplattform nach einem der vorhergehenden Ansprüche, **gekennzeichnet durch** Mittel zur manuellen Eingabe von Steuerbefehlen, vorzugsweise **durch** manuell betätigbare Eingabemittel an einem medizinischen Werkzeug, und/oder
**durch** Mittel zur automatischen Eingabe oder Steuerung der Kinematik (6) über bildgebende Elemente und/oder Sensorelemente,
und/oder
**durch** Mittel zur Sprachsteuerung der Antriebssteuerung (15) bzw. Kinematik (6).

11. Aktorplattform nach einem der vorhergehenden Ansprüche, **dadurch gekennzeichnet, dass** die Antriebssteuerung für eine Nachführungsoptimierung der Kinematik (6) bei nicht bewegtem Endeffektor (12) ausgebildet ist, und dass für diese Nachführungsoptimierung die Kinematik (6) durch die Antriebsteuerung (15) derart steuerbar ist, dass bei nicht bewegtem Endeffektor (12) die einzelnen Bewegungsachsen der Kinematik (6) so eingestellt werden, dass nach der Einstellung jede Bewegungsachse in einem Zustand befindet, aus dem ein möglichst großer Bewegungshub der der jeweiligen Achse zugeordneten Bewegung möglich ist.

## Claims

1. An universal actuator platform for guiding end effectors (12) in minimally invasive interventions, in which the respective end effector (12) is inserted into a body cavity at an entry point,
with a kinematic system (6) comprising at least one interface for attaching at least one end effector (12),
with at least one drive (19) for the kinematic system (6), and with a drive controller (15) for controlling the drive (19) of the kinematic system (6),
wherein the kinematic system (6) comprises means for being mounted on an operating table (1) or on mounting elements provided thereon, for example on rails, and has a closed design that can be sterilized,
**characterized in**
**that** the kinematic system (6) is designed for tilting the end effector (12) by at least 75° referred to a line extending perpendicular to the plane of the entry point, as well as for moving the tilted end effector (12) by 360° around the line extending perpendicular to the plane of the entry point,
**that** the kinematic system (6) consists at least in a section of a material that is neutral to the medium of an imaging device and/or a device for determining the position and/or orientation,
**that** the kinematic system (6) is manually unlockable or separable, and
**that** the drive controller (15) comprises an input (14) for generating nominal values and is designed for compensating mechanical, electrical or temperature-related disturbance variables in the control of the drive (19) of the kinematic system (6) in that the drive controller (15) forms part of a closed control loop in order to achieve high accuracy and reliability, wherein the positioning of the drive (19) is controlled in said control loop by comparing the actual value, which is delivered by at least one sensor and represents the actual position of the kinematic system (6), with a nominal value.

2. The actuator platform according to claim 1, **characterized in that** mechanical means and/or software means (22) for maintaining at least the position and/or orientation of a camera image displayed on a monitor (20) constant or approximately constant are provided if the end effector (12) is designed in the form of a camera or part of a camera and if the kinematic system (6) is designed for rotating the tilted end effector (12) about its axis during the motion around the line extending perpendicular to the plane of the entry point.

3. The actuator platform according to claim 1 or 2, **characterized in that** the kinematic system (6) is manually unlockable or separable on at least one articulation.

4. The actuator platform according to one of the preceding claims, **characterized in that** the kinematic system (6) can be laterally mounted on a side, for example a longitudinal side, of the operating table (1) or on mounting elements provided thereon, for example on rails.

5. The actuator platform according to one of the preceding claims, **characterized in that** the drive controller is designed in such a way that the motion of the at least one end effector (12) is controlled by the drive controller (15) with consideration of a plausibility check and/or within a limited motion space.

6. The actuator platform according to one of the preceding claims, **characterized in**
**that** the drive controller (15) is designed in such a way that the motion of the at least one end effector (12) takes place along an optimal motion path, for example on the shortest paths possible and/or within the smallest motion space possible,
and/or
**that** the nominal value input and/or the control takes place in small increments and easily.

7. The actuator platform according to one of the preceding claims, **characterized in that** the kinematic system (6) or its elements (7, 8, 9, 10, 19) respectively consist of a neutral material at least in a section formed above a plane of a patient support (4).

8. The actuator platform according to one of the preceding claims, **characterized in that** the kinematic system (6) can be mounted on the operating table (1) or the patient support (4) by means of at least one quick-action fastener.

9. The actuator platform according to one of the preceding claims, **characterized in that** the at least one drive (19) of the kinematic system (6) is a fluid drive, preferably a fluid drive with at least one master element and one slave element.

10. The actuator platform according to one of the preceding claims, **characterized by** means for manually inputting control commands, preferably manually actuatable input means on a medical tool,
and/or
by means for automatically inputting or controlling the kinematic system (6) with imaging elements and/or sensor elements
and/or
by means for the voice control of the drive controller (15) and the kinematic system (6).

11. The actuator platform according to one of the preceding claims, **characterized in that** the drive controller is designed for a tracking optimization of the kinematic system (6) while the end effector (12) is stationary, and that for realizing this tracking optimization the kinematic system (6) can be controlled by means of the drive controller (15) such that, while the end effector (12) is stationary, the individual motion axes of the kinematic system (6) are adjusted in such a way that after the adjustment each motion axis is in a state, from which a maximum stroke of the motion associated with the respective axis can be realized.

## Revendications

1. Plateforme actrice universelle, destinée à guider des effecteurs terminaux (12) lors d'interventions à invasion minimale, lors desquelles on introduit l'effecteur terminal (12) dans un point d'entrée d'une cavité corporelle,
avec une cinématique (6) comportant au moins une interface destinée à fixer au moins un effecteur terminal (12),
avec au moins un entraînement (19) pour la cinématique (6), ainsi qu'avec un système de commande (15) d'entraînement, destiné à commander l'entraînement (19) de la cinématique (6),
la cinématique (6) comportant des moyens destinés à la fixation sur une table d'opération (1) ou des éléments de fixation qui s'y trouvent, par exemple sur des rails et présentant une forme de construction close, stérilisable,
**caractérisée**
**en ce que** la cinématique (6) est conçue pour une inclinaison des effecteurs terminaux (12) d'au moins 75° en rapport à l'aplomb sur le plan du point d'entrée, ainsi que pour un déplacement de l'effecteur terminal (12) incliné d'au moins 360° autour de l'aplomb sur le plan du point d'entrée,
**en ce qu'**au moins dans une zone partielle, la cinématique (6) est constituée d'une matière première neutre pour le système du milieu d'un dispositif restituant l'image et/ou déterminant les positions et/ou l'orientation,
**en ce que** la cinématique (6) est déverrouillable ou désolidarisable manuellement et
**en ce que** pour créer une valeur de consigne, le système de commande (15) d'entraînement comporte un système de saisie (14) et est conçu pour une compensation de grandeurs perturbatrices mécaniques, électriques ou dues à la température du système de commande de l'entraînement (19) de la cinématique (6) en ce que pour atteindre une précision et une sécurité élevées, le système de commande d'entraînement (15) est une partie d'un circuit de réglage fermé, dans lequel par une comparaison de la valeur réelle fournie par au moins un capteur et représentant la position effective de la cinématique (6) avec une valeur de consigne, il s'effectue un réglage de la position de l'entraînement (19).

2. Plateforme actrice selon la revendication 1, **caractérisée en ce que** dans le cas d'une conception de l'effecteur terminal (12) sous forme de caméra ou d'une partie d'une caméra et dans le cas d'une conception de la cinématique (6) pour une rotation de l'effecteur terminal (12) incliné autour de son axe, lors du déplacement autour de l'aplomb sur le plan du point d'entrée, des moyens mécaniques et/ou des moyens logiciels (22) sont prévus pour garder constante ou approximativement constante au moins la position et/ou l'orientation d'une image de caméra reproduite sur un écran (20).

3. Plateforme actrice selon la revendication 1 ou 2, **caractérisée en ce que** la cinématique (6) est déverrouillable ou désolidarisable manuellement au moins sur une articulation.

4. Plateforme actrice selon l'une quelconque des revendications précédentes, **caractérisée en ce que** la cinématique (6) est susceptible d'être fixée latéralement sur un côté, par exemple sur un côté longitudinal de la table d'opération (1) ou sur des éléments de fixation qui s'y trouvent, par exemple des rails.

5. Plateforme actrice selon l'une quelconque des revendications précédentes, **caractérisée en ce que** le système de commande d'entraînement est conçu de telle sorte que la commande du déplacement de l'au moins un effecteur terminal (12) s'effectue par le système de commande (15) d'entraînement sous considération d'un contrôle de plausibilité et/ou à l'intérieur d'un espace de déplacement délimité.

6. Plateforme actrice selon l'une quelconque des revendications précédentes, **caractérisée**
**en ce que** le système de commande d'entraînement est conçu de telle sorte que le déplacement de l'au moins un effecteur terminal (12) par le système de commande (15) d'entraînement s'effectue sur une trajectoire de déplacement optimale, par exemple sur des trajets les plus courts possible et/ou à l'intérieur d'un espace de déplacement le plus petit possible
et/ou
**en ce que** la saisie de la valeur de consigne et/ou la commande s'effectue en petites étapes et de manière simple.

7. Plateforme actrice selon l'une quelconque des revendications précédentes, **caractérisée en ce qu'**au moins dans la partie formée au-dessus d'un plan de support du patient (4), la cinématique (6) ou ses éléments (7, 8, 9, 10, 19) est constituée de la matière neutre.

8. Plateforme actrice selon l'une quelconque des revendications précédentes, **caractérisée en ce que** la cinématique (6) est susceptible d'être fixée par une fermeture à serrage rapide sur la table d'opération (1) ou le support du patient (4).

9. Plateforme actrice selon l'une quelconque des revendications précédentes, **caractérisée en ce que** l'au moins un entraînement (19) de la cinématique (6) est un entraînement par fluide, par exemple un tel avec au moins un élément donneur et un élément preneur.

10. Plateforme actrice selon l'une quelconque des revendications précédentes, **caractérisée par** des moyens pour la saisie manuelle d'instructions de commande, de préférence par des moyens de saisie à actionnement manuel sur un instrument médical
et/ou
par des moyens pour la saisie ou commande automatique de la cinématique (6) par l'intermédiaire d'éléments restituant l'image et/ou d'éléments capteurs
et/ou
par des moyens pour la commande vocale du système de commande (15) d'entraînement ou de la cinématique (6).

11. Plateforme actrice selon l'une quelconque des revendications précédentes, **caractérisée en ce que** le système de commande d'entraînement est conçu pour une optimisation de poursuite de la cinématique (6), lorsque l'effecteur terminal (12) n'est pas déplacé et **en ce que** pour cette optimisation de poursuite, la cinématique (6) est susceptible d'être commandée par le système de commande (15) d'entraînement de telle sorte que lorsque l'effecteur terminal (12) n'est pas déplacé, les axes de déplacement individuels de la cinématique (6) se règlent de sorte qu'après le réglage, chaque axe de déplacement se trouve dans une position à partir de laquelle la course de déplacement la plus importante possible du déplacement associé à l'axe concerné est possible.
